# EUROPÄISCHE PATENTANMELDUNG

(11) **EP 3 660 184 A1**
(43) Veröffentlichungstag der Anmeldung: **03.06.2020**
(21) Anmeldenummer: 18209412.8
(22) Anmeldetag: 30.11.2018
(51) Int. Cl.: C23C 16/04, C23C 16/458, H01J 37/32, A61L 27/02, A61F 2/82, A61L 31/08

(54) **HALTERUNGEN ZUR VOLLSTÄNDIGEN PECVD-BESCHICHTUNG VON KÖRPERN, INSBESONDERE STENTS**

(71) Anmelder: BIOTRONIK AG, 8180 Bülach (CH)
(72) Erfinder: Schwarz, Christian, 18069 Rostock (DE); Topp, Anke, 18057 Rostock (DE); Homuth, Torsten, 18211 Ostseebad Nienhagen (DE)
(74) Vertreter: Randoll, Sören

(57) **Zusammenfassung**

Die Erfindung betrifft eine Vorrichtung (1) zum Halten einer Vielzahl an Werkstücken (2), die eine umlaufende Wandstruktur (3) aufweisen, die einen Innenraum (4) des jeweiligen Werkstücks (2) umgibt, wobei die Vorrichtung (1) zum Halten der Werkstücke (2) eine Vielzahl an rotierbaren Halteelementen (10) aufweist, wobei die Halteelemente (10) zum Festlegen der Werkstücke (2) konfiguriert sind. Weiterhin betrifft die Erfindung ein Verfahren zum Beschichten eines Werkstücks (2), insbesondere Stents, unter Verwendung einer erfindungsgemäßen Vorrichtung (1).

## Beschreibung

Die Erfindung betrifft eine Vorrichtung zum Halten von Werkstücken, insbesondere Stents, insbesondere beim Beschichten der Werkstücke bzw. Stents. Derartige Beschichtungen dienen dabei insbesondere der Verbesserung der Biokompatibilität der betreffenden Werkstücke/Stents. Hierzu kann z.B. ein Schichtsystem bestehend aus zumindest zwei Schichten nacheinander aufgetragen bzw. erzeugt werden. Das Schichtsystem weist vorzugsweise eine Schicht aufweisend Si (Silizium) sowie eine weitere Schicht aufweisend SiC (Siliziumcarbid) auf, wobei die Schichten insbesondere durch plasmaunterstützte chemische Gasphasenabscheidung (englisch plasma-enhanced chemical vapour deposition, PECVD) erzeugt werden können.

Aus der WO2016/008687A1 ist eine Halterung bekannt, bei der jeweils ein Stent lose auf einem rotierbaren Adapter angeordnet ist, so dass der jeweilige Stent beim Rotieren des zugeordneten Adapters eine Rotation ausführen kann.

Hiervon ausgehend liegt der vorliegenden Erfindung die Aufgabe zugrunde eine Vorrichtung zum Halten von Werkstücken, insbesondere in Form von Stents, bereitzustellen, die eine vollumfängliche Beschichtung des jeweiligen Werkstücks bei sicherem manuellem Handling und hoher Kapazität ermöglicht.

Diese Aufgabe wird durch eine Vorrichtung mit den Merkmalen des Anspruchs 1 gelöst. Vorteilhafte Ausgestaltungen der Vorrichtung werden in den entsprechenden Unteransprüchen angegeben und nachfolgend im Detail beschrieben.
Gemäß Anspruch 1 wird eine Vorrichtung zum Halten einer Vielzahl an Werkstücken offenbart, wobei die Werkstücke jeweils eine umlaufende Wandstruktur aufweisen, die einen Innenraum des jeweiligen Werkstücks umgibt, und wobei die Vorrichtung zum Halten der Werkstücke eine Vielzahl an rotierbaren Halteelementen aufweist, wobei die Halteelemente zum Festlegen (insbesondere Festklemmen) der Werkstücke konfiguriert sind.

Gemäß einer Ausführungsform der Vorrichtung ist vorgesehen, dass das jeweilige Werkstück ein Stent ist, wobei die Wandstruktur des jeweiligen Stents eine aus einer Vielzahl von Streben gebildete Gitterstruktur ist.

Weiterhin ist gemäß einer Ausführungsform der Vorrichtung vorgesehen, dass das jeweilige Halteelement durch zwei parallele Drahtabschnitte gebildet ist, die dazu konfiguriert sind, sich durch den Innenraum zumindest eines Werkstücks zu erstrecken, derart, dass das mindestens eine Werkstück an den beiden parallelen Drahtabschnitten festgeklemmt ist.

Gemäß einer Ausführungsform ist vorgesehen, dass der jeweilige Drahtabschnitt einen Durchmesser aufweist, der kleiner oder gleich 200 µm ist, insbesondere kleiner oder gleich 100µm. Weiterhin ist gemäß einer Ausführungsform vorgesehen, dass der jeweilige Drahtabschnitt aus einem Metall, insbesondere aus rostfreiem Edelstahl, insbesondere der Sorte 1.4301 gebildet ist.

Weiterhin ist gemäß einer Ausführungsform der Vorrichtung vorgesehen, dass die beiden Drahtabschnitte des jeweiligen Halteelementes - bezogen auf einen Zustand, in dem an dem jeweiligen Halteelement kein Werkstück festgeklemmt ist - zum Festklemmen des mindestens einen Werkstücks einen Abstand senkrecht zu einer Längsachse des jeweiligen Drahtabschnitts aufweisen, der größer ist als der Innendurchmesser des Innenraumes des mindestens einen festzuklemmenden Werkstücks, wobei der Abstand insbesondere zumindest 0,1mm größer ist als der besagte Innendurchmesser.

Weiterhin ist gemäß einer Ausführungsform der Vorrichtung vorgesehen, dass die Vorrichtung dazu ausgebildet ist, das jeweilige Halteelement (bzw. die beiden Drahtabschnitte) um eine zugeordnete Rotationsachse zu rotieren, die parallel zu den Drahtabschnitten des jeweiligen Halteelementes verläuft und sich insbesondere mittig zwischen den beiden Drahtabschnitten des jeweiligen Halteelementes erstreckt.

Gemäß einer weiteren Ausführungsform der Vorrichtung ist vorgesehen, dass die Vorrichtung dazu ausgebildet ist, die Halteelemente (bzw. die jeweiligen beiden Drahtabschnitte) synchron um die jeweilige Rotationsache zu rotieren.

Weiterhin ist gemäß einer Ausführungsform der Vorrichtung vorgesehen, dass die Vorrichtung einen Träger zum Tragen der Halteelemente aufweist, wobei die Haltelemente zwischen einem ersten und einem gegenüberliegenden zweiten Schenkel des Trägers aufgespannt sind bzw. sich zwischen den beiden Schenkeln erstrecken.

Weiterhin ist gemäß einer Ausführungsform der Vorrichtung vorgesehen, dass das jeweilige Halteelement zum Rotieren um seine Rotationsachse mit einem am ersten Schenkel gelagerten ersten Zahnrad und mit einem am zweiten Schenkel gelagerten zweiten Zahnrad gekoppelt ist.

Gemäß einer Ausführungsform der Vorrichtung ist weiterhin vorgesehen, dass je zwei benachbarte erste Zahnräder des ersten Schenkels miteinander kämmen, und/oder dass je zwei benachbarte zweite Zahnräder des zweiten Schenkels miteinander kämmen.

Weiterhin ist gemäß einer Ausführungsform der Vorrichtung vorgesehen, dass die ersten Zahnräder über eine Antriebswelle mit den zweiten Zahnrädern gekoppelt sind, so dass die ersten und zweiten Zahnräder synchron rotierbar sind, um die Halteelemente um die jeweilige Rotationsachse zu rotieren, wobei sich die Antriebswelle parallel zu den Halteelementen erstreckt.

Weiterhin ist gemäß einer Ausführungsform der Vorrichtung vorgesehen, dass die Vorrichtung einen (z.B. am Träger festgelegten) Antrieb zum Rotieren der besagten Antriebswelle aufweist.

Weiterhin ist gemäß einer Ausführungsform der Vorrichtung vorgesehen, dass das jeweilige erste Zahnrad auf einer zugeordneten ersten Welle angeordnet ist, die mit einem ersten Anschlagzylinder verbunden ist, der sich über eine Feder an dem ersten Schenkel abstützt, wobei die jeweilige Feder zum Vorspannen der Drahtabschnitte eines Halteelements konfiguriert ist, und wobei die jeweilige erste Welle mit einem zugeordneten Haken verbunden ist, der zwei gekrümmte Abschnitte zum Aufnehmen eines Drahtverbindungsabschnitts aufweist, der die beiden Drahtabschnitte des betreffenden Halteelements integral miteinander verbindet. Die Drahtabschnitte bilden also zwei miteinander verbundene Stränge eines Drahtes.

Gemäß einer Ausführungsform der Vorrichtung ist weiterhin vorgesehen, dass der jeweilige Haken vorzugsweise W-förmig ausgebildet ist. Die Vorrichtung kann weiterhin als Werkzeug eine separate Nadel aufweisen, die zum Aufsetzen der Drahtverbindungsabschnitte auf den jeweiligen Haken ausgebildet ist.

Weiterhin ist gemäß einer Ausführungsform der Vorrichtung vorgesehen, dass das jeweilige zweite Zahnrad auf einer zugeordneten zweiten Welle angeordnet ist, die mit einem zweiten Anschlagzylinder verbunden ist, der sich an dem zweiten Schenkel abstützt, wobei die beiden Drahtabschnitte des jeweiligen Halteelementes jeweils ein freies Ende aufweisen, wobei die jeweilige zweite Welle mit einem zugeordneten Spannzylinder verbunden ist, wobei der jeweilige Spannzylinder dazu ausgebildet ist, die beiden freien Enden der Drahtabschnitte des betreffenden Halteelementes zu klemmen.

Hierzu kann der jeweilige Spannzylinder für jedes freie Ende eine Kerbe zur Aufnahme des jeweiligen freien Endes eines Drahtabschnitts aufweisen, sowie je eine Klemmschraube zum Klemmen des jeweiligen freien Endes, wenn dieses in seiner zugeordneten Kerbe aufgenommenen ist.

Weiterhin ist gemäß einer Ausführungsform der Vorrichtung vorgesehen, dass das jeweilige Halteelement durch einen Drahtabschnitt gebildet ist, der dazu konfiguriert ist, eine eingeprägte Form zum Klemmen eines Werkstücks anzunehmen, wenn sich der Drahtabschnitt durch den Innenraum des Werkstücks erstreckt, wobei der jeweilige Drahtabschnitt dazu ausgebildet ist, sich beim Überschreiten einer Schwellentemperatur ausgehend von einer gestreckten Form in die besagte eingeprägte Form zu verformem.

Die Drahtabschnitte können separate Drahtabschnitte sein, die z.B. durch separate Drähte gebildet sind. Die Drahtabschnitte können jedoch auch integral miteinander verbunden sein und einen einzelnen Draht bilden.

Die Schwellentemperatur liegt vorzugsweise oberhalb der Raumtemperatur bzw. oberhalb von 30°, aber vorzugsweise unterhalb einer beim Beschichten der Werkstücke herrschenden Prozesstemperatur.

Zum Auffädeln der Werkstücke bzw. der Stents kann der betreffende Drahtabschnitt bzw. Draht in die gestreckte bzw. gerade Form gebracht werden, welche der betreffende Drahtabschnitt bzw. Draht insbesondere bei Raumtemperatur behält. Dieser Vorgang ist reversibel. Vorzugsweise ist der jeweilige Drahtabschnitt aus einer Nickel-Titan-Legierung gebildet, insbesondere aus Nitinol.

Bei der eingeprägten Form handelt es sich gemäß einer Ausführungsform der Vorrichtung um eine der folgenden Formen: eine Wellenform, eine Spiralform, eine Zickzackform.

Weiterhin ist gemäß einer Ausführungsform der Vorrichtung vorgesehen, dass das jeweilige Halteelement durch zwei auseinanderlaufende, von einem Draht abgehende Drahtabschnitte gebildet ist, die dazu konfiguriert sind, zum Festlegen eines Werkstücks mit der Wandstruktur des Werkstücks in Eingriff zu treten.

Weiterhin ist gemäß einer Ausführungsform der Vorrichtung vorgesehen, dass die Vorrichtung eine Vielzahl an rotierbaren Wellen aufweist, die mittels eines Antriebs rotierbar sind, wobei jede Welle mehrere der Halteelemente trägt.

Weiterhin ist gemäß einer Ausführungsform der Vorrichtung vorgesehen, dass das jeweilige Halteelement einen mit einer Welle verbundenen Stift aufweist, der dazu konfiguriert ist, zum Festklemmen eines Werkstücks in den Innenraum des Werkstücks einzugreifen, insbesondere derart, dass das Werkstück einen spitzen Winkel mit der betreffenden Welle einschließt.

Gemäß einer Ausführungsform der Vorrichtung ist weiterhin vorgesehen, dass die mehreren Halteelemente einer Welle übereinander angeordnet sind, so dass entlang der Welle mehrere Werkstücke übereinander an dem jeweiligen Halteelement festklemmbar sind.

Gemäß einer Ausführungsform der Vorrichtung ist weiterhin vorgesehen, dass jeweils eine Mehrzahl an Halteelementen, insbesondere drei Halteelemente, der Halteelemente einer Welle von einem Haltering abstehen, wobei der jeweilige Haltering die zugeordnete Welle in Umfangsrichtung umgreift. Der jeweilige Haltering kann offen ringförmig ausgebildet sein, so dass der Haltering auf die betreffende Welle aufgesteckt werden kann.

Entsprechend können weiterhin eine Mehrzahl (insbesondere drei) Werkstücke in Umfangsrichtung der jeweiligen Welle nebeneinander angeordnet werden.

Weiterhin können die Halteelemente bzw. Halteringe eine Welle so angeordnet sein, dass je zwei in vertikaler Richtung bzw. entlang der Welle benachbarte Halteelemente in Umfangsrichtung der Welle zueinander versetzt angeordnet sind.

Weiterhin ist gemäß einer Ausführungsform der Vorrichtung vorgesehen, dass je zwei Halteelemente durch ein erstes und ein zweites Federelement gebildet sind, wobei die beiden Federelemente jeweils einen ersten hakenförmigen Endabschnitt und einen gegenüberliegenden zweiten hakenförmigen Endabschnitt aufweisen, und wobei die beiden Federelemente jeweils in einer Durchgangsöffnung einer Welle angeordnet sind, so dass die Endabschnitte der Federelemente von der Welle abstehen, wobei der erste hakenförmige Endabschnitt des ersten Federelementes dem ersten hakenförmigen Endabschnitt des zweiten Federelementes entlang der Welle gegenüberliegt, und wobei der zweite hakenförmige Endabschnitt des ersten Federelementes dem zweiten hakenförmigen Endabschnitt des zweiten Federelementes entlang der Welle gegenüberliegt.

Die ersten hakenförmigen Endabschnitte sind nun vorzugsweise dazu konfiguriert, mit der Wandstruktur eines Werkstücks in Eingriff zu treten, um das Werkstück an den beiden Federelementen festzulegen. In gleicher Weise sind vorzugsweise die zweiten hakenförmigen Endabschnitte dazu konfiguriert, mit der Wandstruktur eines weiteren Werkstücks in Eingriff zu treten, um das weitere Werkstück an den beiden Federelementen festzulegen.

Gemäß einer Ausführungsform ist vorgesehen, dass zwei weitere Halteelemente in Umfangsrichtung der Welle versetzt zu den beiden Halteelementen angeordnet sind, wobei die beiden weiteren Halteelemente jeweils durch zwei weitere Federelemente gebildet sind, die in zwei weiteren Durchgangsöffnungen der Welle angeordnet sind, wobei die zwei weiteren Durchgangsöffnungen orthogonal zu den Durchgangsöffnungen der beiden anderen Federelemente verlaufen.

Weiterhin ist gemäß einer Ausführungsform der Erfindung vorgesehen, dass jeweils mehrere Halteelemente bzw. deren Federelemente einer Welle entlang der Welle übereinander bzw. nebeneinander angeordnet sind.

Die oben beschriebenen Halteelemente können jeweils von einem Gehäuse der Vorrichtung umgeben sein, in dem mittels der Vorrichtung ein Plasma erzeugbar ist, um mittels plasmaunterstützter chemischer Gasphasenabscheidung eine Beschichtung auf die (insbesondere rotierenden) Werkstücke bzw. Stents aufzubringen. Die Vorrichtung kann zum Erzeugen der Beschichtung entsprechende Elektroden aufweisen.

Gemäß einem weiteren Aspekt der vorliegenden Erfindung wird ein Verfahren zum Beschichten von Werkstücken unter Verwendung einer erfindungsgemäßen Vorrichtung offenbart, wobei die Werkstücke mittels der Vorrichtung gehalten und rotiert werden und mit einer Beschichtung versehen werden.

Gemäß einer Ausführungsform des Verfahrens ist vorgesehen, dass die Werkstücke mittels plasmaunterstützter chemischer Gasphasenabscheidung mit einer Beschichtung versehen werden, wobei bevorzugt die Beschichtung eine erste Schicht aufweist, die Silizium (Si) aufweist, und wobei die Beschichtung eine weitere, auf die erste Schicht aufgebrachte zweite Schicht aufweist, die Siliziumcarbid (SiC) aufweist. Vorzugsweise bestehen die Werkstücke bzw. Stents, auf die die Beschichtung aufgebracht wird aus Metall.

Mittels der erfindungsgemäßen Vorrichtung ist mit Vorteil eine vollständige sowie allseitige Beschichtung ohne Wechsel der Kontaktierung des jeweiligen Werkstücks bzw. Stents möglich.

Die erfindungsgemäße Vorrichtung ermöglicht dabei weiterhin eine zuverlässige Schichthaftung auf allen Bereichen des Werkstücks/Stents. Weiterhin kann eine Werkstoffbeeinflussung des Grundmaterials des Werkstücks/Stents wie beispielsweise eine Versprödung des Materials durch Einlagerung von Wasserstoff vermieden werden, da die Stents leitend aufgehängt werden. Im Ergebnis erlaubt die erfindungsgemäße Vorrichtung einen erhöhten Durchsatz/Kapazität, insbesondere durch eine bessere Platznutzung in der Vorrichtung. Die Erfindung weist schließlich hinsichtlich der drahtbasierten Halteelemente ein hohes Automatisierbarkeitspotential auf und erlaubt insgesamt eine effiziente Erhöhung der Biokompatibilität der behandelten Werkstücke/Stents.

Im Folgenden sollen Ausführungsform sowie Merkmale der Erfindung anhand der Figuren erläutert werden. Es zeigen:
- Fig. 1: perspektivische Ansichten einer Ausführungsform einer erfindungsgemäßen Vorrichtung mit Halteelementen, die aus Drahtabschnitten gebildet sind;
- Fig. 2: eine schematische Darstellung einer weiteren Ausführungsform einer erfindungsgemäßen Vorrichtung mit einem wellenförmigen Halteelement;
- Fig. 3: eine schematische Darstellung einer weiteren Ausführungsform einer erfindungsgemäßen Vorrichtung mit Halteelementen aus divergierenden Drahtabschnitten;
- Fig. 4: eine perspektivische Ansicht einer weitere Ausführungsform einer erfindungsgemäßen Vorrichtung mit rotierbaren Wellen zum Tragen von Halteelementen, die eine stehende Anordnung der Werkstücke bzw. Stents ermöglichen;
- Fig. 5: eine schematische Darstellung eines Halteelements einer Welle der Vorrichtung gemäß Fig. 4; und
- Fig. 6: eine schematische Ansicht einer Abwandlung der in den Figuren 4 und 5 gezeigten Vorrichtung mit Halteelementen aus Federelementen.

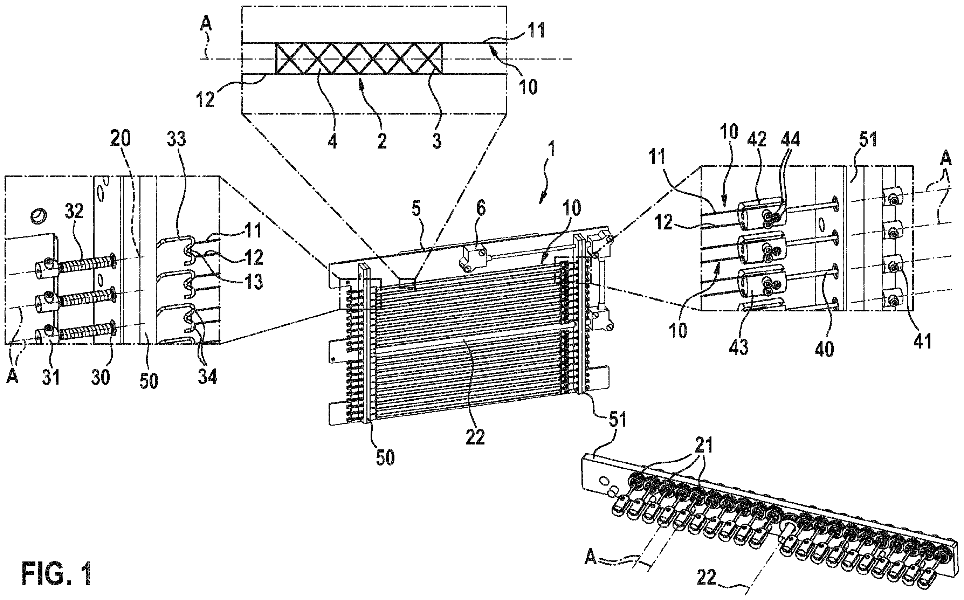

Die vorliegende Erfindung betrifft z.B. gemäß Figur 1 eine Vorrichtung 1 zum Halten einer Vielzahl an Werkstücken 2 die eine umlaufende Wandstruktur 3 aufweisen, die einen Innenraum 4 des jeweiligen Werkstücks 2 umgibt, wobei die Vorrichtung 1 zum Halten der Werkstücke 2 eine Vielzahl an rotierbaren Halteelementen 10 aufweist, wobei die Halteelemente 10 zum Festlegen bzw. zum Festklemmen der Werkstücke 2 konfiguriert sind, wobei es sich bei den Werkstücken 2 vorzugsweise um Stents 2 handelt, wobei die Wandstruktur 3 des jeweiligen Stents 2 insbesondere eine aus einer Vielzahl von Streben gebildete Gitterstruktur ist und entsprechend eine Mehrzahl an Durchgangsöffnungen aufweist. Derartige Stents 2 können z.B. durch Laserschneiden aus einem rohrförmigen Rohling geschnitten werden.

Erfindungsgemäß ist weiterhin verfahrensseitig vorgesehen, eine solche Vorrichtung 1 zum Beschichten von Werkstücken bzw. Stents 2 zu verwenden, wobei die Beschichtung insbesondere mittels PECVD durchgeführt wird und die Vorrichtung 1 zum Halten und insbesondere Rotieren der Werkstücke/Stents 2 bei der Erzeugung der Beschichtung verwendet wird.

Gemäß der in der Figur 1 gezeigten Ausführungsform ist vorgesehen, dass das jeweilige Halteelement 10 durch zwei parallele Drahtabschnitte 11, 12 gebildet ist, die einstückig über einen Drahtverbindungsabschnitt 13 miteinander verbunden sind, und die dazu konfiguriert sind, sich durch den Innenraum 4 zumindest eines Werkstücks bzw. Stents 2 zu erstrecken, derart, dass das mindestens eine Werkstück 2 an den beiden parallelen Drahtabschnitten 11, 12 festgeklemmt ist, indem die Drahtabschnitte 11, 12 vom Innenraum 4 her gegen die Innenseite der Wandstruktur 3 drücken.

Die Vorrichtung gemäß Figur 1 ist weiterhin bevorzugt dazu ausgebildet, das jeweilige Halteelement 10 bzw. die jeweiligen beiden Drahtabschnitte 11, 12 um eine zugeordnete Rotationsachse A zu rotieren, von denen einige in der Figur 1 eingezeichnet sind. Die jeweilige Rotationsachse A verläuft dabei parallel zu den ihr zugeordneten Drahtabschnitten 11, 12 des betreffenden Halteelementes 10 und erstreckt sich dabei insbesondere mittig zwischen den beiden Drahtabschnitten 11, 12.

Weiterhin ist bevorzugt vorgesehen, dass die Vorrichtung einen Antrieb 6 und eine Antriebswelle 22 aufweist, die dazu dienen, die Halteelemente 10 bzw. die jeweiligen beiden Drahtabschnitte 11, 12 synchron um die jeweilige Rotationsache A zu rotieren.

Die Vorrichtung 1 weist weiterhin vorzugsweise einen Träger 5 zum Tragen der Halteelemente 10 sowie insbesondere der Antriebswelle 22 und des Antriebs 6 auf, wobei die Haltelemente 10 gemäß Figur 1 zwischen einem ersten und einem gegenüberliegenden zweiten Schenkel 50, 51 des Trägers 5 aufgespannt sind.

Hierbei ist insbesondere vorgesehen, dass das jeweilige Halteelement 10 zum Rotieren um seine Rotationsachse A mit einem am ersten Schenkel 50 gelagerten ersten Zahnrad 20 (die ersten Zahnräder sind in der Figur 1 nicht sichtbar) und mit einem am zweiten Schenkel 51 gelagerten zweiten Zahnrad 21 gekoppelt ist, wobei je zwei benachbarte erste Zahnräder 20 des ersten Schenkels 50 sowie je zwei benachbarte zweite Zahnräder 21 des zweiten Schenkels 51 miteinander kämmen.

Die ersten Zahnräder 20 sind nun über die besagte Antriebswelle 22 mit den zweiten Zahnrädern 21 gekoppelt, so dass die einander gegenüberliegenden ersten und zweiten Zahnräder 20, 21 synchron rotierbar sind, um die Halteelemente 10 um die jeweilige Rotationsachse A zu rotieren.

Im Einzelnen ist hierbei insbesondere vorgesehen, dass das jeweilige erste Zahnrad 20 auf einer zugeordneten ersten Welle 30 angeordnet ist, die mit einem ersten Anschlagzylinder 31 verbunden ist, der sich über eine Feder 32 an dem ersten Schenkel 50 abstützt, wobei die jeweilige Feder 32 zum Vorspannen der Drahtabschnitte 11, 12 des zugeordneten Halteelements 10 konfiguriert ist. Weiterhin ist die jeweilige erste Welle 30 mit einem zugeordneten Haken 33, wobei der jeweilige Haken 33 zwei gekrümmte Abschnitte 34 zum Aufnehmen des jeweiligen Drahtverbindungsabschnitts 13 aufweist, der die jeweiligen beiden Drahtabschnitte 11, 12 des jeweiligen Halteelements 10 integral miteinander verbindet. Wie anhand der Figur 1 ersichtlich ist, ist der jeweilige Haken 33 vorzugsweise W-förmig ausgebildet.

Weiterhin ist insbesondere vorgesehen, dass das jeweilige zweite Zahnrad 21 auf einer zugeordneten zweiten Welle 40 angeordnet ist, die mit einem zweiten Anschlagzylinder 41 verbunden ist, der sich an dem zweiten Schenkel 51 abstützt. Die beiden Drahtabschnitte 11, 12 des jeweiligen Halteelementes 10 weisen des Weiteren jeweils ein freies Ende auf, wobei die jeweilige zweite Welle 40 mit einem zugeordneten Spannzylinder 42 verbunden ist, wobei der jeweilige Spannzylinder 42 dazu ausgebildet ist, die beiden freien Enden der Drahtabschnitte 11, 12 des zugeordneten Halteelementes 10 zu klemmen. Hierzu kann der jeweilige Spannzylinder 42 für jedes freie Ende eine Kerbe 43 zur Aufnahme des jeweiligen freien Endes eines Drahtabschnitts 11, 12 aufweisen, sowie je eine Klemmschraube 44 zum Klemmen des jeweiligen freien Endes eines Drahtabschnitts 11 bzw. 12, wenn dieses in seiner zugeordneten Kerbe 43 aufgenommenen ist.

Die Vorrichtung 1 erlaubt somit auf effiziente Weise das Auffädeln der Werkstücke 2 auf die Halteelemente 10 bzw. Drahtabschnitte 11, 12. Hiernach können die Halteelemente 10 bzw. deren Drahtverbindungsabschnitte 13 in die Haken 33 eingehängt werden. Die Federelemente 32 erlauben dabei ein Spannen der Halteelemente 10, was gleichzeitig die Werkstücke an den Halteelementen 10 bzw. Drahtabschnitten 11, 12 festlegt.

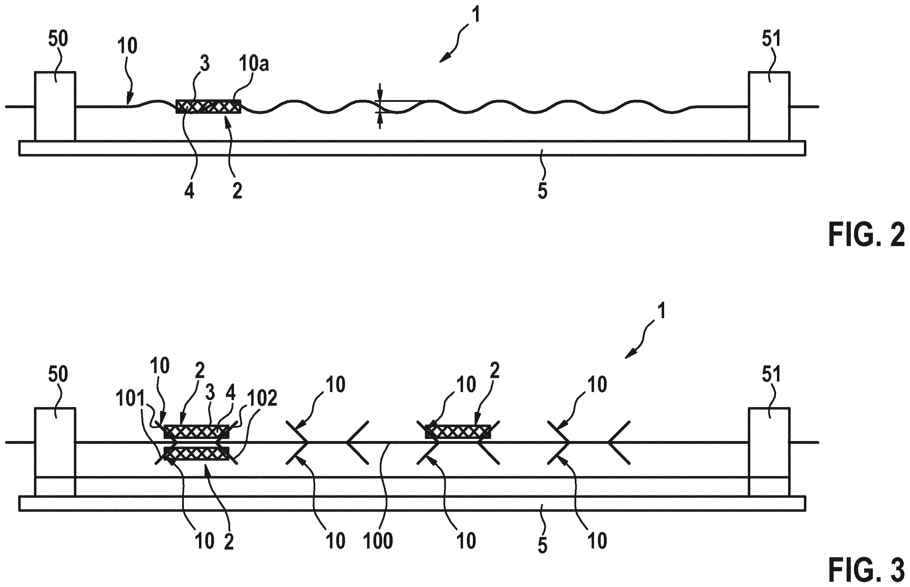

Gemäß einer alternativen Ausführungsform der Vorrichtung 1 ist gemäß Figur 2 vorgesehen, dass das jeweilige Halteelement 10 durch einen Drahtabschnitt 10a gebildet ist, der dazu konfiguriert ist, eine eingeprägte Form zum Klemmen eines Werkstücks 2 reversibel anzunehmen, wenn sich der Drahtabschnitt 10a durch den Innenraum 4 des Werkstücks 2 erstreckt, wobei der jeweilige Drahtabschnitt 10a dazu ausgebildet ist, sich beim Überschreiten einer Schwellentemperatur ausgehend von einer gestreckten Form in die besagte eingeprägte Form zu verformen. Die Schwellentemperatur liegt hierbei vorzugsweise oberhalb der Raumtemperatur bzw. oberhalb von 30°C aber vorzugsweise unterhalb einer beim Beschichten der Werkstücke herrschenden Prozesstemperatur.

Zum Auffädeln der Werkstücke 2 bzw. der Stents 2 kann der betreffende Drahtabschnitt 10a in eine gestreckte bzw. gerade Form gebracht werden, welche der betreffende Drahtabschnitt 10a vorzugsweise bei Raumtemperatur aufweist. Der jeweilige Drahtabschnitt kann z.B. aus einer Formgedächtnislegierung (z.B. eine Nickel-Titan-Legierung, insbesondere Nitinol) gebildet sein. Bei der eingeprägten Form kann es sich z.B. gemäß Figur 2 um eine Wellenform handeln. Andere Formen, die ein Klemmen der Werkstücke 2 erlauben, sind auch denkbar. Auch hier können mehrere Werkstücke 2 hintereinander auf einem Drahtabschnitt 10a bzw. Halteelement 10 aufgefädelt werden.

Weiterhin ist gemäß einer weiteren Ausführungsform der Vorrichtung 1 gemäß Figur 3 vorgesehen, dass das jeweilige Halteelement 10 durch zwei auseinanderlaufende, von einem Draht 100 abgehende Drahtabschnitte 101, 102 gebildet ist, die dazu konfiguriert sind, zum Festlegen eines Werkstücks 2 mit der Wandstruktur 3 des Werkstücks 2 in Eingriff zu treten. Da Stents in der Regel eine Vielzahl an Durchgangsöffnungen aufweisen, ist eine derartiger Aufbau insbesondere zum Halten/Festlegen von Stents 2 geeignet.

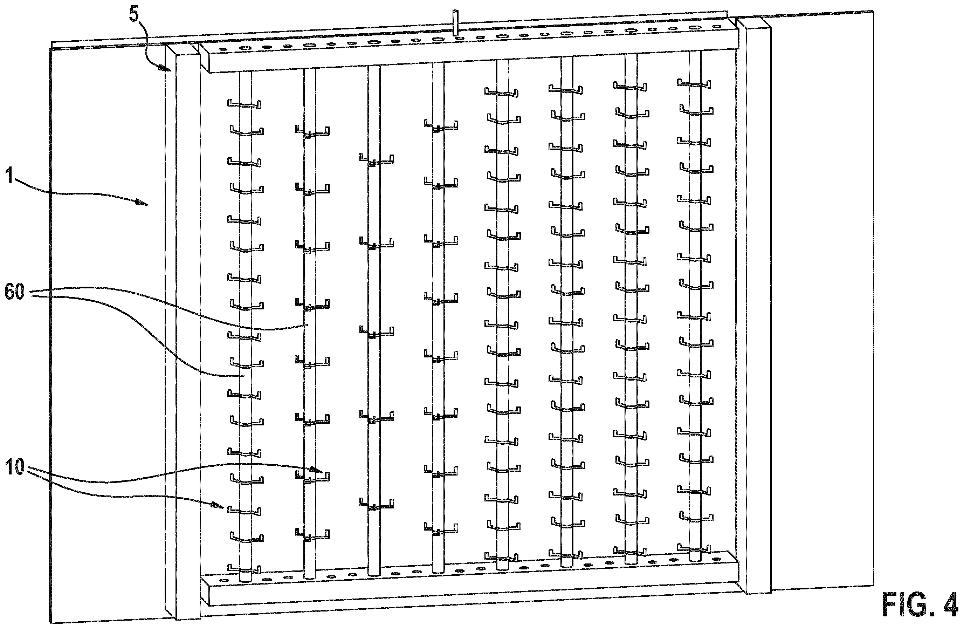

Weiterhin ist gemäß den in den Figuren 4 bis 6 gezeigten Ausführungsformen der Vorrichtung 1 vorgesehen, dass die Vorrichtung zum Rotieren der Halteelemente 10 eine Vielzahl an insbesondere vertikal erstreckten sowie rotierbaren Wellen 60 aufweist, die z.B. mittels eines Antriebs 6 rotierbar sind (z.B. jeweils um eine vertikal erstrecke Rotationsache bzw. um die jeweilige Längsachse der Welle 60), wobei jede Welle 60 vorzugsweise mehrere der Halteelemente 10 trägt.

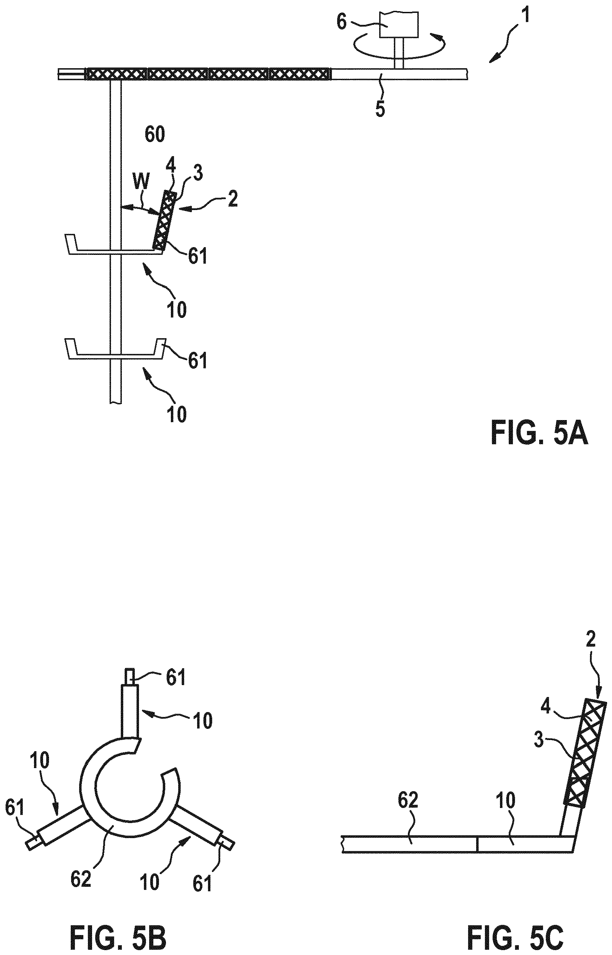

Gemäß der in der Figuren 4 und 5 gezeigten Ausführungsform ist vorgesehen, dass das jeweilige Halteelement 10 einen mit einer Welle 60 verbundenen Stift 61 aufweist (vgl. insbesondere Fig. 5 (C)), der dazu konfiguriert ist, zum Festklemmen eines Werkstücks 2 in den Innenraum 4 des Werkstücks 2 einzugreifen, insbesondere derart, dass das Werkstück 2 einen spitzen Winkel W mit der Welle 60 einschließt (vgl. Fig. 5 (A)). Der jeweilige Stift 61 ist vorzugsweise als flacher Körper ausgebildet, um einen Kontakt mit der Innenseite des Werkstücks/Stents 2 flächenmäßig klein zu halten.

Insbesondere ist vorgesehen, dass die mehreren Halteelemente 10 einer Welle 60 übereinander angeordnet sind (vgl. Fig. 4), so dass entlang der Welle 60 mehrere Werkstücke 2 übereinander an dem jeweiligen Halteelement 10 festklemmbar sind.

Weiterhin ist bevorzugt vorgesehen, dass jeweils eine Mehrzahl an Halteelementen 10, insbesondere drei Halteelemente 10 einer Welle 60 von einem Haltering 62 abstehen (vgl. Fig 5 (B)), wobei der jeweilige Haltering 62 die zugeordnete Welle 60 in Umfangsrichtung der Welle 60 umgreift. Der jeweilige Haltering 62 kann offen ringförmig ausgebildet sein, so dass der Haltering 62 auf die betreffende Welle 60 aufgesteckt werden kann. Entsprechend können weiterhin eine Mehrzahl (insbesondere drei) Werkstücke 2 in Umfangsrichtung der jeweiligen Welle 60 nebeneinander angeordnet werden. Weiterhin können die Halteelemente 10 bzw. Halteringe 62 einer Welle 60 so angeordnet sein, dass je zwei in vertikaler Richtung bzw. entlang der Welle 60 benachbarte Halteelemente 10 in Umfangsrichtung der Welle 60 zueinander versetzt angeordnet sind (vgl. Fig. 4).

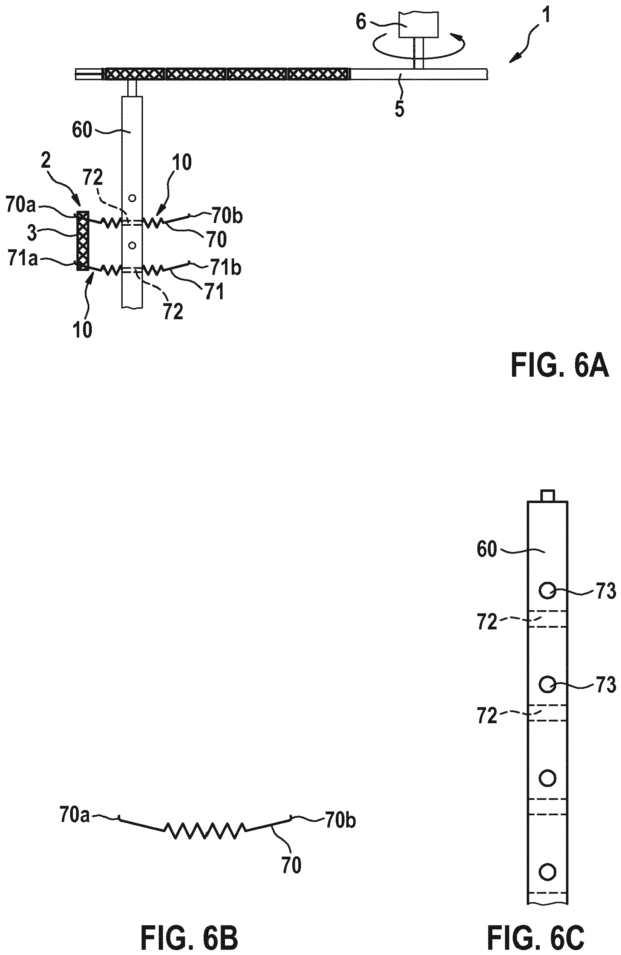

Weiterhin ist alternativ gemäß der in der Figur 6 gezeigten Ausführungsform der Vorrichtung 1 vorgesehen, dass je zwei Halteelemente 10 durch ein erstes und ein zweites Federelement 70, 71 gebildet sind, wobei die beiden Federelemente 70, 71 jeweils einen ersten hakenförmigen Endabschnitt 70a, 71a und einen gegenüberliegenden zweiten hakenförmigen Endabschnitt 70b, 71b aufweisen (vgl. Fig. 6 (A) und (B)), und wobei die beiden Federelemente 70, 71 jeweils in einer Durchgangsöffnung 72 einer Welle 60 angeordnet sind, so dass die Endabschnitte 70a, 70b, 71a, 71b der Federelemente 70, 71 von der Welle 60 bzw. aus der entsprechenden Durchgangsöffnung 72 herausragen, wobei der erste hakenförmiger Endabschnitt 70a des ersten Federelementes 70 dem darunter angeordneten ersten hakenförmigen Endabschnitt 71a des zweiten Federelementes 71 entlang der Welle 60 gegenüberliegt. In gleicher Weise liegt der zweite hakenförmige Endabschnitt (70b) des ersten Federelementes 70 dem darunter angeordneten zweiten hakenförmigen Endabschnitt 71b des zweiten Federelementes 71 entlang der Welle 60 gegenüber, wobei die ersten hakenförmigen Endabschnitte 70a, 71a dazu konfiguriert sind, mit der Wandstruktur 3 eines Werkstücks/Stents 2 in Eingriff zu treten, um das Werkstück bzw. den Stent 2 an den beiden Federelementen 70, 71 festzulegen, und wobei die zweiten hakenförmigen Endabschnitte 70b, 71b dazu konfiguriert sind, mit der Wandstruktur 3 eines weiteren Werkstücks bzw. Stents 2 in Eingriff zu treten, um das weitere Werkstück/Stent 2 an den beiden Federelementen 70, 71 festzulegen.

Vorzugsweise ist weiterhin vorgesehen (vgl. insbesondere Fig. 6 (C)), dass zwei weitere Halteelemente in Umfangsrichtung der Welle 60 versetzt zu den beiden Halteelementen 10 bzw. 70, 71 angeordnet sind, wobei die beiden weiteren Halteelemente jeweils durch zwei weitere Federelemente gebildet sind, die in zwei weiteren Durchgangsöffnungen 73 der Welle 60 angeordnet sind, wobei die zwei weiteren Durchgangsöffnungen 73 orthogonal zu den Durchgangsöffnungen 72 der beiden anderen Federelemente 70, 71 verlaufen. Weiterhin können natürlich mehrere Halteelemente 10 bzw. 70, 71 einer Welle 60 entlang der Welle 60 übereinander bzw. nebeneinander angeordnet sein.

Die oben beschriebenen Halteelemente 10 können jeweils von einem Gehäuse der Vorrichtung 1 umgeben sein, in dem mittels der Vorrichtung 1 ein Plasma erzeugbar ist, um z.B. mittels plasmaunterstützter chemischer Gasphasenabscheidung eine Beschichtung auf die (insbesondere rotierenden) Werkstücke 2 bzw. Stents 2 aufzubringen.

## Patentansprüche

1. Vorrichtung (1) zum Halten einer Vielzahl an Werkstücken (2) die eine umlaufende Wandstruktur (3) aufweisen, die einen Innenraum (4) des jeweiligen Werkstücks (2) umgibt, wobei die Vorrichtung (1) zum Halten der Werkstücke (2) eine Vielzahl an rotierbaren Halteelementen (10) aufweist, wobei die Halteelemente (10) zum Festlegen der Werkstücke (2) konfiguriert sind.

2. Vorrichtung nach Anspruch 1, **dadurch gekennzeichnet, dass** das jeweilige Werkstück (2) ein Stent ist, wobei die Wandstruktur (3) des jeweiligen Stents (2) eine aus einer Vielzahl von Streben gebildete Gitterstruktur ist.

3. Vorrichtung nach Anspruch 1 oder 2, **dadurch gekennzeichnet, dass** das jeweilige Halteelement (10) durch zwei parallele Drahtabschnitte (11, 12) gebildet ist, die dazu konfiguriert sind, sich durch den Innenraum (4) zumindest eines Werkstücks (2) zu erstrecken, derart, dass das mindestens eine Werkstück (2) an den beiden parallelen Drahtabschnitten (11, 12) festgeklemmt ist.

4. Vorrichtung nach einem der vorhergehenden Ansprüche, **dadurch gekennzeichnet, dass** die Vorrichtung (1) dazu ausgebildet ist, das jeweilige Halteelement (10) um eine Rotationsachse (A) zu rotieren, die jeweils parallel zu den Drahtabschnitten (11, 12) des jeweiligen Halteelementes (10) verläuft und sich insbesondere mittig zwischen den beiden Drahtabschnitten (11, 12) des jeweiligen Halteelementes (10) erstreckt.

5. Vorrichtung nach einem der vorhergehenden Ansprüche, **dadurch gekennzeichnet, dass** die Vorrichtung (1) einen Träger (5) zum Tragen der Halteelemente (10) aufweist, wobei die Haltelemente (10) zwischen einem ersten und einem gegenüberliegenden zweiten Schenkel (50, 51) des Trägers (5) aufgespannt sind.

6. Vorrichtung nach Anspruch 5, **dadurch gekennzeichnet, dass** das jeweilige Halteelement (10) zum Rotieren um seine Rotationsachse (A) mit einem am ersten Schenkel (50) gelagerten ersten Zahnrad (20) und mit einem am zweiten Schenkel (51) gelagerten zweiten Zahnrad (21) gekoppelt ist.

7. Vorrichtung nach Anspruch 6, **dadurch gekennzeichnet, dass** die ersten Zahnräder (20) über eine Antriebswelle (22) mit den zweiten Zahnrädern (21) gekoppelt sind, so dass die ersten und zweiten Zahnräder (20, 21) synchron rotierbar sind, um die Halteelemente (10) um die jeweilige Rotationsachse (A) zu rotieren, wobei sich die Antriebswelle (22) parallel zu den Halteelementen (10) erstreckt.

8. Vorrichtung nach Anspruch 6 oder 7, **dadurch gekennzeichnet, dass** das jeweilige erste Zahnrad (20) auf einer ersten Welle (30) angeordnet ist, die mit einem ersten Anschlagzylinder (31) verbunden ist, der sich über eine Feder (32) an dem ersten Schenkel (50) abstützt, wobei die jeweilige Feder (32) zum Vorspannen der Drahtabschnitte (11, 12) eines Halteelements (10) konfiguriert ist, und wobei die jeweilige erste Welle (30) mit einem Haken (33) verbunden ist, der zwei gekrümmte Abschnitte (34) zum Aufnehmen eines Drahtverbindungsabschnitts (13) aufweist, der die beiden Drahtabschnitte (11, 12) eines Halteelements (10) integral miteinander verbindet.

9. Vorrichtung nach einem der Ansprüche 6 bis 8, **dadurch gekennzeichnet, dass** das jeweilige zweite Zahnrad (21) auf einer zweiten Welle (40) angeordnet ist, die mit einem zweiten Anschlagzylinder (41) verbunden ist, der sich an dem zweiten Schenkel (51) abstützt, wobei die beiden Drahtabschnitte (11, 12) des jeweiligen Halteelementes (10) jeweils ein freies Ende aufweisen, wobei die jeweilige zweite Welle (40) mit einem Spannzylinder (42) verbunden ist, wobei der jeweilige Spannzylinder (42) dazu ausgebildet ist, die beiden freien Enden der Drahtabschnitte (11, 12) eines Halteelementes (10) zu klemmen.

10. Vorrichtung nach Anspruch 1 oder 2, **dadurch gekennzeichnet, dass** das jeweilige Halteelement (10) durch einen Drahtabschnitt (10a) gebildet ist, der dazu konfiguriert ist, eine eingeprägte Form zum Klemmen eines Werkstücks (2) anzunehmen, wenn sich der Drahtabschnitt (10a) durch den Innenraum (4) des Werkstücks (2) erstreckt, wobei der jeweilige Drahtabschnitt (10a) dazu ausgebildet ist, sich beim Überschreiten einer Schwellentemperatur ausgehend von einer gestreckten Form in die besagte eingeprägte Form zu verformen.

11. Vorrichtung nach Anspruch 1 oder 2, **dadurch gekennzeichnet, dass** das jeweilige Halteelement (10) durch zwei auseinanderlaufende, von einem Draht (100) abgehende Drahtabschnitte (101, 102) gebildet ist, die dazu konfiguriert sind, zum Festlegen eines Werkstücks (2) mit der Wandstruktur (3) des Werkstücks (2) in Eingriff zu treten.

12. Vorrichtung nach Anspruch 1 oder 2, **dadurch gekennzeichnet, dass** die Vorrichtung zum Rotieren der Halteelemente (10) eine Vielzahl an rotierbaren Wellen (60) aufweist, die mittels eines Antriebs (6) rotierbar sind, wobei jede Welle (60) mehrere der Halteelemente (10) trägt.

13. Vorrichtung nach Anspruch 12, **dadurch gekennzeichnet, dass** das jeweilige Halteelement (10) einen mit einer Welle (60) verbundenen Stift (61) aufweist, der dazu konfiguriert ist, zum Festklemmen eines Werkstücks (2) in den Innenraum (4) des Werkstücks (2) einzugreifen.

14. Vorrichtung nach Anspruch 12, **dadurch gekennzeichnet, dass** je zwei Halteelemente (10) durch ein erstes und ein zweites Federelement (70, 71) gebildet sind, wobei die beiden Federelemente (70, 71) jeweils einen ersten hakenförmigen Endabschnitt (70a, 71a) und einen gegenüberliegenden zweiten hakenförmigen Endabschnitt (70b, 71b) aufweisen, und wobei die beiden Federelemente (70, 71) jeweils in einer Durchgangsöffnung (72) einer Welle (60) angeordnet sind, so dass die Endabschnitte (70a, 70b, 71a, 71b) der Federelemente (70, 71) von der Welle (60a) abstehen, wobei der erste hakenförmige Endabschnitt (70a) des ersten Federelementes (70) dem ersten hakenförmigen Endabschnitt (71a) des zweiten Federelementes (71) entlang der Welle (60) gegenüberliegt, und wobei der zweite hakenförmige Endabschnitt (70b) des ersten Federelementes (70) dem zweiten hakenförmigen Endabschnitt (71b) des zweiten Federelementes (71) entlang der Welle (60) gegenüberliegt, wobei die ersten hakenförmigen Endabschnitte (70a, 71a) dazu konfiguriert sind, mit der Wandstruktur (3) eines Werkstücks (2) in Eingriff zu treten, um das Werkstück (2) an den beiden Federelementen (70, 71) festzulegen, und wobei die zweiten hakenförmigen Endabschnitte (70b, 71b) dazu konfiguriert sind, mit der Wandstruktur (3) eines weiteren Werkstücks (2) in Eingriff zu treten, um das weitere Werkstück (2) an den beiden Federelementen (70, 71) festzulegen.

15. Verfahren zum Beschichten von Werkstücken (2) unter Verwendung einer Vorrichtung (1) nach einem der vorhergehenden Ansprüche, wobei die Werkstücke (2) mittels der Vorrichtung (1) gehalten und rotiert werden und mit einer Beschichtung versehen werden.
